# EUROPEAN PATENT APPLICATION

(11) **EP 4 480 407 A1**
(43) Date of publication of application: **25.12.2024**
(21) Application number: 23020299.6
(22) Date of filing: 19.06.2023
(51) Int. Cl.: A61B 5/12, A61B 5/00

(54) **A SYSTEM, DEVICE AND METHOD FOR MONITORING EARDRUM IMPEDANCE**

(71) Applicant: Zeta Diagnostics ApS, 8200 Aarhus N (DK)
(72) Inventor: Linde, Kasper, DK8330 Beder (DK); Villadsen, Naja, 8250 Lystrup (DK); Friis, Christian, DK8800 Viborg (DK); Nijkamp, Jasper Albertus, DK8240 Risskov (DK)
(74) Representative: Weinrich, Søren Gert

(57) **Abstract**

Those who suffer from eustachian tube dysfunction do not all experience symptoms continuously. The pressure of the middle ear may be normal most of the time, but temporary problems may arise at other times.

Therefore, a tympanometry system is configured to monitor the functional acoustic characteristics of the ear drum as a function of time by measuring and monitoring the acoustic impedance of the ear drum as a function of time over a monitoring period based on a measurement sound signal emitted by a speaker (4) and a corresponding sound signal detected by a microphone (6). Display means (20') are configured to display the acoustic impedance or related acoustic quantity as a function of time. The system may further comprise storage means configured to store one or more target acoustic impedance as a function of time, and during the monitoring period compares the measured acoustic impedance with a corresponding target acoustic impedance, determines the difference (22) therebetween, and displays (20) and/or stores (30) said difference as a function of time on suitable display means (20') and/or in suitable storage means (30).

## Description

### TECHNICAL FIELD

The present invention relates generally to a system comprising an ear-mounted measurement device and associated controller and more specifically to such a system designed to measure the physiological function of the eustachian tube of a human being.

### BACKGROUND OF THE INVENTION

The human ear is comprised of an ear canal which leads into the ear and is separated from the middle ear (ME), inside the user's head, by an eardrum. The middle ear is normally maintained at a pressure close to ambient pressure and thereby properly biases the eardrum such that a substantially undistorted transmission of soundwaves to the inner ear is obtained. Furthermore, pressure differences in the middle ear in the order of +/- 100 dap relative to ambient pressure have been shown to be an important driver of the development of middle ear diseases.

The middle ear is an air-filled cavity in the human head, which is ventilated with ambient air through the eustachian tube (ET). The ET orifices in the rear of the pharynx and when the ET opens, a small amount of ambient air is transferred into the middle ear through the ET. Consequently, the partial pressures of nitrogen, carbon dioxide and oxygen are higher in the ME cavity than in the peripheral blood. As a result, a negative pressure will always form, due to the diffusion of gasses from the ME cavity into the peripheral blood passing the ME cavity in its mucosal lining. Two important structures are believed to balance this ever-forming negative pressure:
1) The eustachian tube (ET) regulates the pressure of the middle ear by opening periodically to transfer a small amount of air into the middle ear; and
2) the mucosal lining of the air cells within the mastoid bone is believed to have some sort of buffer capacity due to the ability to either swell or contract.

Eustachian tube dysfunction (ETD) is a dynamic disease where the eustachian tube is malfunctioning and thus unable to serve as the important pressure valve created by evolution.

As ETD is a dynamic condition where the ET may only be periodically malfunctioning, combined with the fact that one can have the condition for a number of years with no symptoms, diagnosing ETD is a difficult and cumbersome task. ETD often results in a prolonged time with a pathological negative ME-pressure being maintained in the middle ear, causing the eardrum to be sucked into the ME, thus increasing the tension of the eardrum. This can lead to discomfort, loss of hearing, chronic infections and an arsenal of severe middle ear disorders.

Changes of the pressure in the middle ear causes changes in the acoustic impedance of the ear drum because the pressure difference causes changes of the tension on the ear drum. The popping in one's head that one hears when yawning, swallowing or in an environment of changing pressure like an airplane is related to the opening of the eustachian tube controlling the pressure of the middle ear. The ear comprises many other key features which provide for the full capabilities of hearing that will not be described further here.

Existing solutions of assessing the functioning of the middle ear and the eustachian tube are based on measurements with a tympanometer.

A tympanometer is generally a handheld device that is inserted into the ear canal of a patient by a medical practitioner. During a pressure sweep, a measurement soundwave is output and a reflection of the measurement soundwave is detected. Based on the detected reflected soundwave, the tympanometer determines the acoustic impedance of the eardrum and, based on the acoustic impedance, can make a determination of the pressure in the middle ear.

Tympanometry is a test which is used to assess the status of the eardrum and the health of the middle ear. It does so by providing a snapshot of the middle ear pressure along with the compliance of the ear drum.

However, those who suffer from eustachian tube dysfunction do not all experience symptoms continuously. Instead, the pressure of the middle ear may be normal most of the time, but temporary problems may arise at other times. This can be problematic for detecting and diagnosing eustachian tube dysfunction using a tympanometer, because it may be the case that an acoustic impedance measurement performed by a medical practitioner with a tympanometer will be performed when there is no problem and, as such, the measurement will provide no diagnosis of eustachian tube dysfunction.

### OBJECTIVES OF THE INVENTION

On the above background it is an objective of the present invention to provide a system or a device that can monitor the functional acoustic characteristics of the ear drum as a function of time.

It is a further objective of the present invention to provide a system or device that is adaptable to a specific ear thereby increasing the accuracy of the monitoring of the acoustic characteristics of the ear drum.

It is a further objective of the present invention to provide a system or device that can minimize the annoyance of a long monitoring period for the person whose acoustic characteristics of the eardrum is being monitored.

### DISCLOSURE OF THE INVENTION

The above objectives and further advantages are obtained by the various aspects of the invention defined below.

According to a first aspect of the invention there is provided a system comprising a measurement device wherein the measurement device is configured to be mounted to the ear of a user, the user's ear having an ear canal and an ear drum, the measurement device comprising:
- a speaker configured to emit a sound signal into the ear canal as part of a measurement of the acoustic impedance or a related quantity such as the acoustic admittance or reflectance of the ear drum;
- an internal microphone configured to detect sound signals in the ear canal; and
- a securing apparatus configured to provide for securing of the measurement device to the ear,
wherein the system further comprises a controller configured to measure and monitor the acoustic impedance or a related acoustic quantity such as the acoustic admittance or reflectance of the ear drum as a function of time over a monitoring period based on a measurement sound signal emitted by the speaker and a corresponding sound signal detected by the microphone, and display means configured to display the acoustic impedance or related acoustic quantity as a function of time.

In one or more embodiments of the first aspect the system is provided with storage means configured to store one or more target acoustic impedance or related acoustic quantity as a function of time, and during the monitoring period comparing the measured acoustic impedance or related acoustic quantity as a function of time with a corresponding target acoustic impedance or related acoustic quantity as a function of time and determining the difference between the determined acoustic impedance or related acoustic quantity as a function of time and the corresponding target as a function of time and displaying and/or storing said difference as a function of time on suitable display means and/or in suitable storage means.

In one or more embodiments of the first aspect the system comprises means for providing a calibration audio signal to the ear drum, where the calibration audio signal is configured to allow determining a measurement frequency or measurement frequency band, which is suitable or preferably even optimal for determining the acoustic impedance or the related acoustic quantity.

In one or more embodiments of the first aspect the calibration audio signal as a chirp covering the frequency range in which the acoustic impedance or related acoustic quantity is to be measured.

In one or more embodiments of the first aspect the system comprises means for providing a masking audio signal, the acoustic characteristics of which enables it to wholly or partially mask the measurement audio signal, and where the system during the monitoring period in addition to the measurement audio signal at the measurement frequency band or measurement frequency band also emits the masking audio signal, such that a person whose ear drum characteristics are being monitored during the monitoring period is less annoyed by the measurement signal and hence will be more able to accept a prolonged monitoring period.

In one or more embodiments of the first aspect, the controller is configured to interpret changes of the acoustic impedance or a related acoustic quantity of the ear drum as an opening of the eustachian tube and the controller is configured to record the number of eustachian tube openings over the monitoring period.

In one or more embodiments of the first aspect, the controller is configured to determine one or more additional properties of the opening of the eustachian tube wherein the changes of the acoustic impedance of the ear drum are further indicative of the one or more additional properties of the opening of the eustachian tube.

In one or more embodiments of the first aspect, the one or more additional properties of the opening of the eustachian tube comprise one or more of:
a duration of a eustachian tube opening
a time between eustachian tube openings; and
the shape of the plot of eustachian tube opening as a function of time for instance in order to ascertain that the plot in fact represents an opening of the eustachian tube and not for instance random noise.

In one or more embodiments of the first aspect, the system is configured to monitor the acoustic impedance of the ear drum over the monitoring period by being configured to one or more of:
emit a sound signal continuously over at least a portion of the monitoring period, wherein the continuously emitted sound signal is emitted for a sufficient duration to resolve at least two measurements of the acoustic impedance of the ear drum;
emit a plurality of successive sound signals over at least a portion of the monitoring period having a predetermined delay period between each successive emitted sound signal; and wherein the controller is configured to one or more of:
continuously determine the acoustic impedance of the ear drum for long enough to resolve at least two measurements of the acoustic impedance of the eardrum; or
determine the acoustic impedance of the eardrum a plurality of individual times, wherein each acoustic impedance determination is based on the reflections of successively emitted sound signals having a predetermined delay period therebetween.

In one or more embodiments of the first aspect, the portion of the monitoring period is at least 30 seconds.

In one or more embodiments of the first aspect, the system is configured to monitor the acoustic impedance of the ear drum over the monitoring period and the system is configured to emit a sound signal continuously over at least a portion of the monitoring period, wherein the continuously emitted sound signal is a signal having such spectral and temporal characteristics that it is able to either totally or partially mask the measurement signal, such that the person whose ear drum characteristics are being monitored is less annoyed by the measurement signal and hence will be more able to accept a prolonged monitoring period.

In one or more embodiments of the first aspect, the system is configured to provide an alert based on the acoustic impedance of the ear drum changing by more than a predetermined acceptable threshold amount.

In one or more embodiments of the first aspect, the emitted sound signal comprises a single tone sound signal.

In one or more embodiments of the first aspect, the speaker is configured to emit sound signals at the measurement frequency or in the measurement frequency band according to a predetermined schedule.

In one or more embodiments of the first aspect, the controller is configured to receive trigger signalling from a user input device and wherein, on receipt of the trigger signalling, the controller is configured to perform an acoustic impedance measurement or a measurement of the related acoustic quantity.

In one or more embodiments of the first aspect, the controller is configured to compare audio detected by the internal microphone to one or more audio profiles stored in an audio profile database and, based on identification of a match of the detected audio to an audio profile, the controller is configured to record the identification of the match. In such embodiments, the audio signal profiles of the audio signal profile database comprise (but is not limited to) one or more of the list comprising: a yawn action; a swallow action; a breathing action; a coughing action, a Toynbee maneuverer or a Valsalva maneuverer. When a match is found, the time of the occurrence may be stored in the system, for instance in a datalogger, thereby making it possible clearly to assess if a corresponding pattern in a plot of eardrum impedance versus frequency really is caused by an impedance change or is the result of the patient for instance yawning or coughing.

In an embodiment of the first aspect, the controller is configured to provide signalling to a user-interface device to cause the user-interface device to provide instructions to a user about how to perform a manoeuvre.

In an embodiment of the first aspect, the ear-mounted measurement device further comprises a sealing device configured to seal the ear canal.

In one or more embodiments of the first aspect, recording the identification of the audio match comprises recording the fact that a match occurred.

In one or more embodiments of the first aspect, recording the identification of the match further comprises recording the type of match that occurred.

In one or more embodiments of the first aspect, recording the identification of the match further comprises recording the time that the match occurred.

In one or more embodiments of the first aspect, the controller is further configured to correlate the timing of the identification of the match with a time when one or more a eustachian tube openings occur.

In one or more embodiments of the first aspect, the identification of a match in an audio profile is used in determining the functioning of a eustachian tube.

In one or more embodiments of the first aspect, the system is configured to perform an acoustic impedance measurement based on identification of a match of the detected audio to an audio profile.

In one or more embodiments of the first aspect, the audio profiles of the audio profile database correspond to one or more of the the list comprising, but not limited to: a yawn action; a swallow action; a breathing action; a Toynbee maneuverer or a Valsalva maneuverer.

In one or more embodiments of the first aspect, the controller is configured to provide signalling to a user-interface device to cause the user-interface device to provide instructions to a user about how to perform a maneuverer.

In one or more embodiments of the first aspect, the ear-mounted measurement device further comprises a sealing device configured to seal the ear canal.

In one or more embodiments of the first aspect the ear-mounted measurement device further comprises an external microphone configured to detect audio signals in the environment around the user and wherein the controller is configured to use the audio signals detected by the external microphone to compensate for audio signals detected by the internal microphone originating external to the ear canal.

In one or more embodiments of the first aspect the ear-mounted measurement device is a first ear-mounted measurement device and the system further comprises a second ear-mounted measurement device wherein the second ear-mounted measurement device is for mounting to a second ear of the user, different from the ear in which the first ear-mounted measurement device is mounted, and wherein the second ear-mounted measurement device comprises a contra-lateral microphone, wherein the controller is configured to compare audio detected by the contra-lateral microphone to one or more audio profiles of an audio profile database and, based on identification of a match of the audio to an audio profile, the controller is configured to record the identification of the match.

In one or more embodiments of the first aspect the system is configured to perform an acoustic impedance measurement based on identification of a match of the detected audio from the contra-lateral microphone to an audio profile.

In one or more embodiments of the first aspect, the ear-mounted measurement device further comprises an external microphone configured to detect audio in the environment around the user and wherein the controller is configured to use the audio detected by the external microphone to compensate for audio detected by the internal microphone originating external to the ear canal.

In one or more embodiments of the first aspect, the ear-mounted measurement device is a first ear-mounted measurement device and the system may further comprise a second ear-mounted measurement device wherein the second ear-mounted measurement device is for mounting to a second ear of the user, different from the ear in which the first ear-mounted measurement device is mounted to and wherein the second ear-mounted measurement device comprises a contra-lateral microphone, wherein the controller is configured to compare audio detected by the contra-lateral microphone to one or more audio profiles of an audio profile database and, based on identification of a match of the audio to an audio profile, the controller is configured to record the identification of the match.

In one or more embodiments of the first aspect, the system is configured to perform an acoustic impedance measurement based on identification of a match of the detected audio from the contra-lateral microphone to an audio profile.

In one or more embodiments of the first aspect, the ear-mounted measurement device is a first ear-mounted measurement device and the system further comprises a second ear-mounted measurement device wherein the second ear-mounted measurement device is for mounting to a second ear of the user, different from the ear to which the first ear-mounted measurement device is mounted and wherein the second ear-mounted measurement device comprises:
a speaker configured to emit a soundwave into the ear canal of the second ear as part of an auxiliary acoustic impedance measurement;
an internal microphone configured to detect soundwaves in the ear canal of the second ear; and
a securing apparatus configured to provide for securing of the second ear-mounted measurement device to the second ear,
wherein the controller is configured to determine an acoustic impedance of the ear drum of the second ear as part of the auxiliary acoustic impedance measurement and compare the acoustic impedance of the ear drum of the first ear and the acoustic impedance of the ear drum of the second ear.

In one or more embodiments of the first aspect, the ear-mounted measurement device further comprises an accelerometer and wherein the accelerometer is configured to detect a movement of the ear and wherein the controller is configured to record the detection of the movement of the ear.

In one or more embodiments of the first aspect, the controller is configured to compare movement detected by the accelerometer to one or more movement profiles stored in a movement profile database and, based on identification of a match of the movement to a movement profile, the controller is configured to record the identification of the match.

In one or more embodiments of the first aspect, the controller is configured to correlate the timing of the identification of the movement match with a time when one or more a eustachian tube openings occur.

In one or more embodiments of the first aspect, the identification of a match of detected movement to a movement profile is used in determining the functioning of a eustachian tube.

In one or more embodiments of the first aspect, the system is configured to perform an acoustic impedance measurement based on identification of a match of the detected movement to a movement profile.

According to a second aspect of the present invention, there is provided an ear-mounted measurement device configured to be mounted to the ear of a user, the user's ear having an ear canal and an ear drum, the ear-mounted measurement device comprising at least:
a speaker configured to emit a soundwave at a measurement frequency into the ear canal as part of an acoustic impedance measurement;
an internal microphone configured to detect soundwaves in the ear canal; and
a securing apparatus configured to provide for securing of the ear-mounted measurement device to the ear,
wherein the ear-mounted measurement device is configured to provide signalling to a controller indicative of the soundwaves detected by the internal microphone for recording the number of eustachian tube openings over a monitoring by monitoring the acoustic impedance of the ear drum over the monitoring period.

According to a third aspect of the present invention, there is provided a controller configured to:
provide signalling to an ear-mounted measurement device to cause a speaker of the ear-mounted measurement device or in the system according to the first aspect to emit a soundwave at a measurement frequency as part of an acoustic impedance measurement;
receive signalling from a microphone of the ear-mounted measurement device indicative of audio detected by the microphone;
monitor the acoustic impedance of the ear drum over a monitoring period based on the signalling indicative of the audio detected by the microphone as part of the acoustic impedance measurement wherein changes of the acoustic impedance of the ear drum are indicative of the opening of the eustachian tube; and
record the number of eustachian tube openings over the monitoring period.

According to a fourth aspect of the present invention there is provided a method for measuring the acoustic impedance or a related acoustic quantity such as the acoustic admittance or reflectance of the ear drum of a patient as a function of time over a monitoring period, the method comprising the steps of:
- providing an ear-mounted measurement device configured to be mounted to the ear of a user, the user's ear having an ear canal and an ear drum, the ear-mounted measurement device comprising at least a speaker configured to emit a sound signal into the ear canal and an internal microphone configured to detect sound signals in the ear canal;
- via the ear canal providing a calibration audio signal to the ear drum, where the calibration audio signal is configured to allow determining an individual measurement frequency or measurement frequency band which is suitable for determining the acoustic impedance or the related acoustic quantity;
- during a monitoring period, providing a measurement sound signal at the individual measurement frequency or frequency band to the ear canal of the patient a plurality of times during the monitoring period, whereby the acoustic impedance or related acoustic quantity is measured a corresponding plurality of times during the monitoring period;
- storing and/or displaying the measured acoustic impedance or related acoustic quantity as a function of time.

In one or more embodiments of the fourth aspect the calibration audio signal is a chirp covering the frequency range in which the acoustic impedance or related acoustic quantity is to be made.

In one or more embodiments of the fourth aspect the speaker in the ear-mounted measurement device during the monitoring period in addition to the signal at the measurement frequency band or measurement frequency band also emits an audio signal, the acoustic characteristics of which enables it to wholly or partially mask the measurement audio signal.

The various aspects of the present invention will de described in the detailed description of the invention by means of a non-limiting example embodiment of the invention. It is however understood that the invention can be implemented in practice in many other ways that what is shown in the figures and described in the detailed description of the invention and that all such implementations fall within the scope defined by the independent claims.

### BRIEF DESCRIPTION OF THE DRAWINGS

One or more embodiments will now be described by way of example only with reference to the accompanying drawings in which
figure 1 shows an example embodiment of a measurement device according to the invention;
figure 2 shows a cross sectional view of the embodiment shown in figure 1;
figure 3 shows schematic examples of plots of ear drum impedance as a function of time illustrating the problem that is solved by the present invention;
figure 4 shows a block diagram of an example embodiment of a system according to the invention, and
figure 5(a) and 5(b) show a flowchart illustrating an embodiment of the method according to the invention.

### DETAILED DESCRIPTION OF THE INVENTION

In its basic form, the system according to the invention comprises a controller and associated data analysis means and data storage and retrieval means. The system further comprises an ear-mounted measurement device. By providing an ear-mounted measurement device, acoustic impedance measurements can be taken according to a plurality of different measurement regimes over an extended period of time without a user having to visit a medical practitioner on a regular basis. A system according to the present disclosure can provide a measurement and monitoring of the pressure regulating function or dynamic function of a eustachian tube by monitoring the number of eustachian tube openings and, optionally, other parameters of the eustachian tube over time. That is, the physiological status of the eustachian tube can be monitored.

The system may be a single device which integrates both the controller and the ear-mounted measurement device into a single unit which can be mounted to the ear of a user. In other examples, the system may be a distributed device comprising an ear-mounted measurement device and a separate controller that is incorporated into a computing device remote from the ear-mounted measurement device. In yet other embodiments, a first portion of the controller configured to carry out certain functionalities may be incorporated in a first device, such as the ear-mounted measurement device, and a second portion of the controller configured to carry out certain other functionalities may be incorporated into a second device, such as a remote computing device.

The controller may be any appropriate electronic controller that is configured to provide signalling to and receive signalling from the ear-mounted measurement device or any component thereof. The controller may comprise at least one processor and at least one memory including computer program code. The at least one memory and the computer program code may be configured to, with the at least one processor, cause the controller to provide for signalling to the ear-mounted measurement device. The controller may also be configured to receive signalling from the ear-mounted measurement device and process the received signalling such that the controller can act on the information comprised within the signalling. The actions that the controller can take based on the received signalling will be described in greater detail below.

It will be appreciated that any suitable means may be used by the controller to provide for the provision and receipt of signalling. For example, the controller may be electrically coupled to the ear-mounted measurement system such that signals are transmitted directly via physical conducting means such as wires, conductive tracks, conductive fibres or otherwise. Alternatively, the controller may provide for signalling to cause a wireless transceiver to provide for transmission and receipt of signalling to the ear-mounted measurement device and the ear-mounted measurement device may comprise a corresponding transceiver configured to both transmit and receive signalling. In such examples, the ear-mounted measurement device may comprise a measurement device controller (not shown) configured to interpret the signalling received from the controller. In embodiments where the controller is remote from the ear-mounted measurement device, the controller and the ear-mounted measurement device may be configured to provide signalling therebetween for communication by any suitable wireless communication technique, such as via Bluetooth, a wi-fi network or another communication means. The controller may be configured to provide for some or all of the data processing associated with the identification of a compliance of the ear drum during an acoustic impedance measurement.

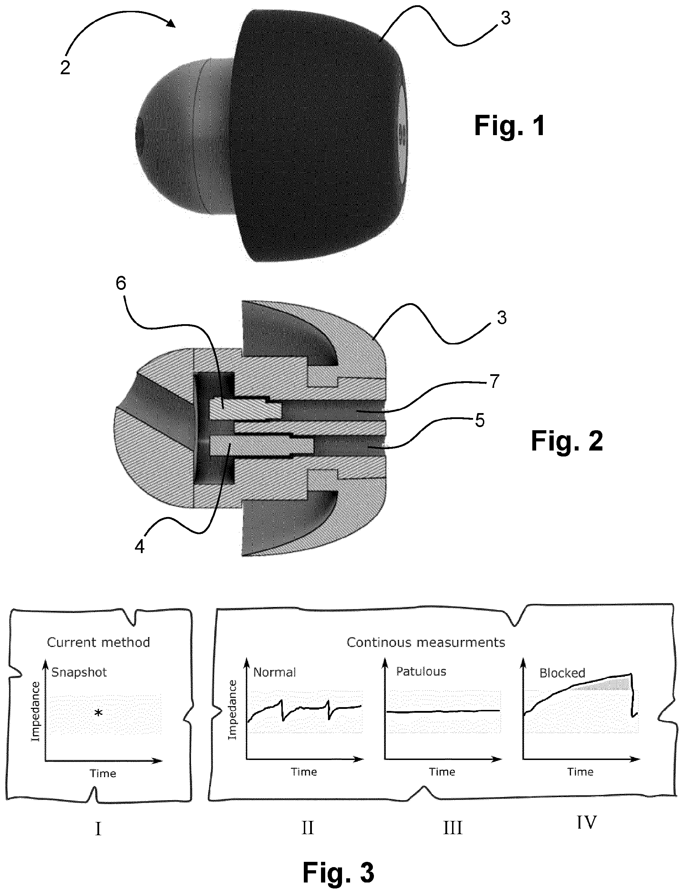

With reference to figure 2 there is shown an example embodiment of an ear-mounted measurement device 2 having a soft portion 3 for insertion into the ear canal.

With reference to figure 3 there is shown a cross section of the example embodiment of the ear-mounted measurement device shown in figure 2. By providing an ear-mounted measurement device configured to be mounted to the ear of a user, the device can be worn for an extended period of time, thereby allowing for continuous, periodic or triggered measurements of the acoustic impedance of the ear drum to be performed. In this way, a user does not need to visit a medical practitioner in order to check for eustachian tube dysfunction and a hand-held measurement device does not need to be used at precisely the right time. Instead, the measurements can be recorded for showing to a medical practitioner or the measurements may be sent automatically to the medical practitioner for evaluation.

The ear-mounted measurement device 2 may be configured to be mounted to the ear of a user in any suitable manner by way of a securing apparatus. For example, the securing apparatus may comprise an ear-hook which is configured to extend over and around the ear in order to hold the ear-mounted measurement device 2 in place. In other examples, the securing apparatus may be an earbud, such as a resiliently deformable earbud, which is configured to be inserted into and held within the ear by way of frictional forces. In this embodiment, the earbud may provide the functionality of both a sealing device and a securing apparatus.

In some embodiments, there may be provided a sealing device, which may be of any suitable shape and made of any suitable material to provide a seal to the ear canal. The sealing device may be configured to substantially prevent the ingress or egress of air in or out of the ear canal. The seal may be sufficient such that a pressure may be applied to the ear drum wherein said pressure is different to the ambient pressure around the user's head. The seal may or may not substantially reduce soundwaves from sources external to the ear canal from being heard by the user. It will be appreciated that anything inserted into the ear canal will reduce the amplitude of soundwaves incident on the ear drum but that some ear buds, for example, are configured to specifically provide for noise blocking. It will be appreciated that pressurising the ear drum may be achieved by applying pressure-applying soundwaves wherein the pressure-applying soundwaves may be of suitably low frequency to apply a pressure to the ear drum which does not oscillate at a frequency that would interfere with emitted measurement signals. Applying pressure in this way may provide a time-varying pressure gradient within the ear canal but the controller may be configured to control a speaker 4 that in the shown embodiment is in acoustic communication with the ear canal via a tube 5 of the ear-mounted measurement device 2 such that the low frequency soundwaves apply pressure to the ear drum when an acoustic impedance measurement is made. Applying pressure to the ear using the speaker 4 may provide for an improved acoustic impedance measurement. In other embodiments, the ear-mounted measurement device may comprise an air pump which is configured to, based on signalling from the controller, pump air into the ear canal in order to pressurise the ear canal. The pump may be used in embodiments with or without the sealing apparatus, however, it will be appreciated that the pump may be most efficiently employed where the sealing apparatus is in place.

The speaker 4 of the ear-mounted measurement device 2 is configured to emit a sound signal (a sound wave) at a measurement frequency into the ear canal of a user as part of an acoustic impedance measurement. The speaker 4 may be arranged inside the ear-mounted measurement device 2 such that it points into the ear canal so that it can emit soundwaves into the ear canal in a substantially unimpeded manner.

The speaker 4 may receive an output signal from the controller configured to cause the speaker 4 to output one or more soundwaves at frequencies defined by the controller. One of these frequencies may be the measurement frequency, where the measurement frequency is either a predetermined frequency or a frequency obtained by a calibration sequence. One example of a signal that could advantageously be used in a calibration sequence is a chirp, i.e., a sinusoidal signal in which the frequency change as a function of time from a given lowest frequency to a given highest frequency. The measurement frequency may after the calibration sequence be selected as a frequency which is particularly suitable to obtain a good quality acoustic impedance measurement. An indicator of a good measurement quality may be a large change in impedance when the middle ear pressure changes and such a large change of impedance may be induced for measurements by the use of a Valsalva manoeuvre, for example, and compared to ambient pressure measurements. The emitted sound signal may be a single tone sound signal, that is, a sound signal of a fixed substantially single frequency. For example, the single tone sound signal may have a frequency of between 20 - 20,000 Hz. In other embodiments, the sound signal may have a frequency of between 20 - 3,000 Hz. In other examples, the tone may be substantially 1.000 Hz.

The speaker 4 may further be configured to emit a pressurising sound signal wherein the pressuring sound signal has a lower frequency than the measurement frequency and wherein the pressurising frequency is selected such that it provides for pressurisation of the ear drum while the sound signal at the measurement frequency is incident on the ear drum. In other embodiments, the ear-mounted measurement device 2 may comprise an air pump configured to apply air to the ear canal such that, when sealed with a sealing device, a pressure in the ear canal is formed in order to bias the ear drum away in a direction in towards the user's head. Regardless of the manner in which pressure is applied, the pressure-applying apparatus, such as the speaker 4 or the pump, may be configured to provide a constant pressure to the ear drum or to apply a time-varying pressure to the ear drum. A time-varying pressure may be useful for allowing the ear-mounted measurement device to additionally perform a traditional tympanometry measurement.

The ear-mounted measurement device 2 further comprises an internal microphone 6 that in the shown embodiment is in acoustic communication with the ear canal via a tube 10 configured to detect soundwaves in the ear canal. The internal microphone 6 may also be arranged inside the ear-mounted measurement device 2 such that it points into the ear canal so as to detect sound signals in the ear canal in a substantially unimpeded manner. In one or more embodiments, the internal microphone 6 may be disposed substantially adjacent the speaker 4 within the ear-mounted measurement device 2. By way of its arrangement, the internal microphone 6 may be configured to detect sound signals which are reflected from the ear drum when the ear-mounted measurement device 2 is mounted to the user's ear in a substantially unimpeded manner. In particular, the microphone 6 may be arranged to detect sound waves reflected from the ear drum, such as reflections of the sound signals emitted by the speaker 4. It is also possible that the internal microphone 6 will detect sound signals originating from outside the ear canal, however, because the sealing device provides for sealing of the ear canal, these sound waves may be distorted as compared to how they may be detected outside of the ear canal. The internal microphone 6 may also detect soundwaves originating from within the user's body such as, but not limited to, the sound of the user swallowing, breathing or yawning. The internal microphone 6 may be configured to provide signalling to the controller 101 indicative of the detected soundwaves.

The controller may be configured to determine the acoustic impedance of the ear drum based on the detected soundwaves as part of the acoustic impedance measurement. In particular, the controller may determine the acoustic impedance of the ear drum based on the signalling received from the internal microphone 6 indicative of the sound signals detected by the microphone 6. The acoustic impedance measurement may comprise the minimum steps of emitting a sound signal at the measurement frequency into the ear canal and the controller determining the acoustic impedance of the ear drum based on the detected soundwaves, that is, the soundwaves detected by the internal microphone 6 which include reflected sound signals. In some embodiments, the controller may not determine the acoustic impedance of the ear drum but may, instead, determine the pressure of the middle ear. The acoustic impedance of the ear drum is a measure of the resistance of the ear drum to the transmission of sound therethrough. The compliance of the ear drum may also be measured and relates to the flexibility of the ear drum and indicates how effectively sound is transmitted into the middle ear.

By providing a measurement device 2 which is mounted to the ear of the user, a plurality of measurements of the acoustic impedance of the ear drum, or similarly, the pressure of the middle ear can be made and the acoustic impedance of the ear drum can be monitored over a monitoring period which is an extended period compared to single measurements of middle ear pressure made by tympanometers. From the plurality of measurements of acoustic impedance, the number and quality of eustachian tube openings can be measured and recorded by controller. The number of eustachian tube openings over the monitoring period may be compared to an expected number of eustachian tube openings that are to be expected for the user or for a user similar to the user. For example, average eustachian tube opening profiles may be used for comparison which comprise averaged or amalgamated data for the opening of a eustachian tube for a healthy eustachian tube. Thus, if the number of recorded eustachian tube openings is within a threshold margin of the eustachian tube opening profile, then this may be an indicator that the function of the user's eustachian tube is healthy. If the number of recorded eustachian tube openings is greater than or smaller than the threshold margin of the eustachian tube opening profile, this may be an indication that the user has eustachian tube dysfunction. Alternatively, or additionally, the recorded eustachian tube openings may be compared to a dis-functioning eustachian tube openings profile which comprises averaged or amalgamated data for the number of openings over a monitoring period from a person with eustachian tube dysfunction.

In addition to, or alternatively to, recording the number of eustachian tube openings, the controller may be configured to record one or more additional properties of the openings of the eustachian tube. Similarly, to counting the number of eustachian tube openings, the acoustic impedance (and, particularly, the changes in the acoustic impedance) of the ear drum may also be indicative of one or more of these additional properties of the openings of the eustachian tube. The controller may be configured to record these properties and either the controller, another device or a remote person, such as a medical practitioner, may compare the recorded properties to expected property profiles for each property where a property profile may comprise averaged or amalgamated information related to that property for either a person with a correctly functioning eustachian tube or a dys-functioning eustachian tube. The additional properties may, alone or together, provide for an indication of the quality of the eustachian tube openings, i.e., whether the eustachian tube is opening properly or in some improper manner. The additional properties of the eustachian tube openings may comprise a delay between two eustachian tube openings. This may be a particularly useful property to measure, as it may give information related to how long the middle ear is in under pressure. Another additional property of the eustachian tube openings may comprise whether a specific action takes place immediately or in close relation to the opening such as but not limited to yawning, swallowing or coughing. Yet another additional property of the eustachian tube openings may be whether a eustachian tube opening results in a full pressure equalisation or a partial pressure equalisation. This may be particularly useful, as it may indicate additional information as to what is the cause of the possible dysfunction.

Providing for a system as described, the user is able to walk around the world as normal and the system can take one or more sets of measurements over one or more monitoring periods over the course of 10 minutes, an hour, 12 hours, 24 hours or another time period.

In some embodiments, the system may make acoustic impedance measurements continuously by causing the speaker to continuously emit sound signals into the ear such as for a duration long enough to resolve at least two measurements of the acoustic impedance of the ear drum. The controller may then be configured to continuously determine the acoustic impedance of the ear drum for long enough to resolve at least two measurements of the acoustic impedance of the ear drum. The time to resolve a single measurement may be dependent on a number of factors including the frequency of the measurement signal used. It will be appreciated that, in this embodiment, the internal microphone 6 may be equally configured to provide for continuous detection of sound signals and may provide signalling indicative of the detected sound signals to the controller. The continuous sound signals may be emitted for a minimum duration of 30 seconds, 1 minute, 5 minutes, 10 minutes, 30 minutes, 1 hour, 2 hours or 6 hours, for example. By providing for this continuous monitoring over at least a portion of the monitoring period, the system may detect eustachian tube function by measuring the number of eustachian tube openings over the monitoring period. The number of eustachian tube openings can, immediately or at a later time, be compared to an expected number of eustachian tube openings in order to identify whether the user is experiencing eustachian tube dysfunction. It may also be possible to determine the quality of the eustachian tube openings and compare these to each other or compare them to an expected quality of eustachian tube opening. In this context, the term "quality" means the shape of the plot of an eustachian tube opening as a function of time compared to an expected shape including for instance a range of shapes bounded by given limits. By determining the quality of the eustachian tube opening, it could for instance be ascertain that the measured plot in fact represents an opening of the eustachian tube and not for instance random noise.

Alternatively, a medical practitioner may take the data recorded by the controller and determine the function (or dysfunction) of the eustachian tube. This is significantly more advantageous than relying on co-incidentally correct timing of an acoustic impedance measurement performed by a handheld tympanometer. Further, in this way, the system may allow for the health or a severity of the eustachian tube dysfunction to be determined by providing an indication of the degree to which the number of eustachian tube openings differed from the expected number of eustachian tube openings.

In other embodiments, the system may make a plurality of acoustic impedance measurements by causing the speaker 4 to emit a plurality of successive sound signals having a predetermined delay period between each successive emitted sound signal. Each emitted sound signal may be a single complete cycle of the waveform or it may comprise a plurality of cycles prior to the delay period. The delay period may be a time period when the speaker 4 provides no output of sound signals. In other examples, the delay period may be a time period when the speaker 4 does not output the emitted signal at the measurement frequency, i.e., the measurement signal for an acoustic impedance measurement. For example, the speaker 4 may be configured to output signals at other frequencies such as for the provision of continuing to provide pressure to the ear or the speaker 4 may be configured to play other audio when not performing an acoustic impedance measurement, such as music or spoken word. The predetermined delay period may be at least 5 seconds, 30 seconds, 1 minute, 5 minutes, 10 minutes 30 minutes, 1 hour, 2 hours, 6 hours or another delay time. As in the case for performing continuous measurements, the provision of periodic acoustic impedance measurements may provide a device which can detect changes in the acoustic impedance of the ear drum, thereby allowing the controller to determine whether eustachian tube openings are occurring in an expected number. The controller can achieve this by comparing a number of detected eustachian tube openings to an expected number of eustachian tube openings. The controller may then determine if the recorded number of eustachian tube openings is below (or above) a healthy or normal number of openings over the course of the monitoring period. This may reduce the dependence on good timing on the part of a hand-administered tympanometry measurement. Further, by only performing periodic acoustic impedance measurements, the experience may be more comfortable for a user because the continuous application of soundwaves to a user's ear over an extended period may otherwise be at least partially discomforting.

In one or more embodiments, on the detection that the recorded number of eustachian tube openings of the ear drum is below or above an expected or desired number of eustachian tube openings, the controller may be configured to control the speaker 4 such that the delay period is reduced so that more regular measurements are taken. Alternatively, on detection that the acoustic impedance of the ear drum is above or below an expected or desired acoustic impedance, the controller may be configured to control the speaker 4 such that the delay period is reduced so that more regular measurements are taken. This may allow the controller to monitor the number of eustachian tube openings during a period that is suspected of being a period of abnormal pressure in the middle ear and, therefore, obtain a better indication of the function of the eustachian tube. Alternatively, on detection that the middle ear is at an abnormal pressure or that the number of eustachian tube openings differs from an expected number of eustachian tube openings, the controller may be configured to change the operating mode of the ear-mounted measurement device such that it provides for continuous acoustic impedance measurements by continuously emitting soundwaves at the measurement frequency as previously discussed. Continuous acoustic impedance measurements may continue to be performed until the number of eustachian tube openings matches an expected number of eustachian tube openings over a time period, at which point, the controller may be configured to adjust the operation mode of the ear-mounted measurement device 2 to once more perform periodic acoustic impedance measurements.

In some embodiments, the predetermined delay between successive emitted soundwaves for the acoustic impedance measurement may not be the same between each successive soundwave. For example, a first delay period may be provided between a first pair of soundwave emissions while a different second delay period may be provided between a second pair of soundwave emissions. In some embodiments, the successive emission of soundwaves may be performed according to a predetermined schedule which may vary the delays between successive soundwave emissions. The schedule may provide for more acoustic impedance measurements at certain times of day compared to other times of day and may be configured to adapt to a user's daily routine.

In yet other examples, the controller may be configured to provide signalling to the ear-mounted measurement device 202 to cause the speaker 205 to emit a soundwave at the measurement frequency continuously for a sufficient duration to resolve at least two measurements of the acoustic impedance of the ear drum and, after said continuous period sufficient for at least two measurements of the acoustic impedance of the ear drum, provide a delay period before performing another continuous acoustic impedance measurement. In this way, continuous measurements over sub-periods of the monitoring period may be taken periodically (with gaps in between) in comparison to single periodic measurements of acoustic impedance. This may provide advantages associated with both continuous and periodic measurements.

In one or more embodiments, the system may be configured to receive trigger signalling from a user input device and, on receipt of the trigger signalling, the controller may be configured to perform an acoustic impedance measurement by at least providing signalling to cause the emission of a soundwave at the measurement frequency and the determination of the acoustic impedance of the ear drum by the controller 101 based on soundwaves detected by the internal microphone 4. If the system 100 is operating in a mode, that causes acoustic impedance measurements to be performed in accordance with a schedule, the receipt of signalling from a user input device may cause the controller to override the scheduling or perform a measurement outside of the scheduled acoustic impedance measurements prior to returning to the schedule. This may be particularly helpful for a user who may be wearing the ear-mounted measurement device for an extended period because, rather than relying on a schedule, the user can provide the user input to cause an acoustic impedance measurement when their ear feels uncomfortable or in pain. The positive identification of an abnormal pressure of the middle ear, which may made based on the number of eustachian tube openings over the monitoring period not equalling an expected number of eustachian tube openings, may provide for a positive diagnosis of eustachian tube dysfunction whereas repeated indications that the number of eustachian openings does match an expected number of eustachian tube openings may provide an indication that eustachian tube is functioning correctly. The user input device may be any suitable device which allows the user to indicate that they wish an acoustic impedance measurement to be made such as a button on the ear-mounted measurement device, an input on a separate controller device, a mobile telephone, tablet computer, computer or other electronic user device.

In one or more embodiments, the controller may be configured to compare audio detected by the internal microphone 6 to one or more audio profiles stored in an audio profile database. It will be appreciated that audio here relates to soundwaves or collection of soundwaves such as that detected by a microphone. The audio profiles may comprise a combination of at least two of time, amplitude (audio volume) and frequency data which together define an audio profile representative of a sound. The audio profiles may correspond to sounds made by the user's body or to sounds expected to be heard in the environment of the user. The audio profiles may particularly relate to sounds made by the human body which are associated with eustachian tube openings. More specifically, the audio profiles may relate to, for example, a yawn action, a swallow action, taking a breath, a Toynbee maneuverer or a Valsalva maneuverer. Alternatively, the audio profile may correspond to an audio command by the user such as a spoken command of "Take measurement". The audio profile database may be a memory comprising one or more audio profiles which are accessible by the controller and which the controller can use to perform comparisons with detected soundwaves or overall detected audio. The audio profiles stored in the audio profile database may be generated by recording sample audio tracks using a microphone such as the internal microphone 6 or another microphone. In other examples, the audio profiles may be generalised audio profiles may be generated by a machine learning algorithm based on a plurality of recorded audio tracks associated with the sound of interest. For example, a machine learning algorithm may listen to a plurality of sample audio tracks of a person swallowing and, based on that plurality of audio tracks, the machine learning algorithm may be configured to pick out the common features of the audio recordings in order to generate a generalised audio profile of a swallowing action. The number and timing of eustachian tube openings expected during any of the actions associated with an audio profile may vary depending on the audio profile and may be expected to occur at particular points during the action. As such, each audio profile may have an expected eustachian tube opening profile associated with it. Each expected eustachian tube opening profile for an action provides a number of eustachian tube openings and, optionally, one or more other parameters relating to the eustachian tube openings which can be compared to the recorded number of eustachian tube openings and one or more other parameters of those openings. For example, the eustachian tube opening profile for an action may comprise an association with a particular part of the audio profile where a eustachian tube opening is expected to occur or it might have a duration of a eustachian tube opening associated with it.

Based on an identification of a match of detected audio to an audio profile, the controller may be configured to record the identification of the match. Recording the identification of the audio match may comprise recording the fact that a match occurred. For example, each time an audio event is recorded, a tally may be increased by one to represent the number of matched audio events. Recording the identification of the match may further comprise recording the type of match that occurred. For example, the number of swallow actions recorded over a monitoring period may be recorded. This number may be compared to, or otherwise used in conjunction with, the record of the number of eustachian tube openings over a monitoring period. This may help in characterising the functioning of the eustachian tube. Recording the identification of the match may further comprise recording the time that the match occurred. For example, the controller may be configured to correlate the timing of the identification of the match with a time when one or more a eustachian tube openings occur. In one or more embodiments, the identification of a match in an audio profile may be used in determining the functioning of a eustachian tube.

Based on an identification of a match of detected audio to an audio profile, the system may further be configured to perform an acoustic impedance measurement, a continuous acoustic impedance measurement or a plurality of acoustic impedance measurements. A match between the detected audio and an audio profile may relate to a match of one or more audio features of the detected audio and the audio profile. For example, the match may be a match in distribution of detected frequencies with respect to time which maps out the changes in frequency over time associated with a sound profile of a swallow action.

Providing for the triggering of an acoustic impedance measurement based on a match between detected audio and an audio profile may be particularly advantageous because it might be expected that a change in the pressure of the middle ear will occur alongside certain actions, such as, but not limited to, a swallow, yawn, taking a breath, a Toynbee maneuverer or Valsalva maneuverer and, as such, one or more eustachian tube openings may be expected that can be compared to expected numbers of eustachian tube openings. This may allow acoustic impedance measurements to be taken as a change in pressure of the middle ear is expected to occur or immediately after an expected change in pressure. By monitoring the acoustic impedance of the ear drum during such actions, the occurrence of eustachian tube openings can be monitored. The presence and number of eustachian tube openings during any of the listed maneuverers can be compared to an expected number of openings during such actions in order to provide a measure of the function of the eustachian tube. The expected number of eustachian tube openings for a particular action may be recorded in a eustachian tube openings profile associated with that action. Alternatively, or additionally, the system may be configured to perform an acoustic impedance measurement after each audio match and, as such, a medical practitioner may obtain a significant amount of data indicative of the eustachian tube function of the user while limiting potential discomfort of a user to only those times when abnormal pressure of the middle ear or acoustic impedance of the ear drum is expected.

The ear-mounted measurement device may further comprise an external microphone arranged such that it points toward the environment around the user and not directly into the ear canal of the user. The external microphone may be arranged particularly such that it detects audio in the environment around the user. That is, the external detects soundwaves originating from outside of the ear canal in a substantially unimpeded manner. The external microphone may be configured to provide signalling to the controller indicative of the audio detected by the external microphone. The controller may be configured to use the audio detected by the external microphone to compensate for external audio detected by the internal microphone as a result of vibrations of the user's ear in response to external soundwaves. This audio compensation may provide for an improved acoustic impedance measurement when in noisy environments.

In one or more embodiments, the ear-mounted measurement device 2 discussed up to this point may be a first ear-mounted measurement device 2 and the system may comprise a second ear-mounted measurement device. The second ear-mounted measurement device may be for mounting to a second ear of the user, different from the ear to which the first ear-mounted measurement device 2 is mounted. The second ear-mounted measurement device may comprise a contra-lateral microphone. The contra-lateral microphone may be configured to particularly detect sounds from within the user's head, such as swallow actions, yawn actions or Valsalva maneuverers. The controller may be configured to compare audio detected by the contra-lateral microphone to one or more audio profiles of an audio profile database. Based on an identification of a match of detected audio to an audio profile by the second ear-mounted measurement device, the controller may be configured to record the identification of the match. Based on identification of a match of the audio detected by the contra-lateral microphone and an audio profile of the audio profile database, the system may further be configured to perform an acoustic impedance measurement or to monitor the acoustic impedance of the ear drum over a monitoring period. The audio detected by the contra-lateral microphone may be used in addition to the audio detected by the internal microphone 6 for determining a match of detected audio to an audio profile. It will be appreciated that the same audio profile database may be used for the contra-lateral microphone as is used for the internal microphone of the first ear-mounted measurement device. Alternatively, the contra-lateral microphone may be configured to compare detected audio to different audio profiles in order to provide for detection of different events. Correlation between the internal microphone 6 of the first ear-mounted measurement device 2 and the contra-lateral microphone may reduce instances of false-positive detections of audio profiles such as swallow actions or yawn actions. Further, audio measurements made by the contra-lateral microphone may be used to filter background noise from measurements taken by the internal microphone of the first ear-mounted measurement device. This may provide for an improvement in the quality of the acoustic impedance data recorded over the monitoring period.

The second ear-mounted measurement device may comprise a securing apparatus configured to provide for securing of the second ear-mounted measurement device to the second ear and the contra-lateral microphone. In some embodiments, the second ear-mounted measurement device may also comprise a sealing device configured to seal the ear canal of the second ear. The second ear-mounted measurement device may further comprise a speaker configured to emit a sound signal at a measurement frequency into the ear canal as part of an auxiliary acoustic impedance measurement. The auxiliary acoustic impedance measurement may also be performed over a monitoring period. The auxiliary acoustic impedance measurement performed on the second ear by the second ear-mounted measurement device may be performed in order to provide a comparison of a healthy ear (the second ear, in this case) to an ear under observations for eustachian tube dysfunction (the first ear, in this case). For example, the number of eustachian tube openings recorded over the auxiliary acoustic impedance measurement performed on the second ear may be compared to the number of eustachian tube openings recorded as part of the acoustic impedance measurement performed on the first ear. This may be performed instead of or in addition to comparing to a predetermined expected number of eustachian tube openings. In other examples, the auxiliary acoustic impedance measurement may additionally be used to assess the function of the second ear. In one or more embodiments, the contra-lateral microphone may be arranged to point inwards toward the ear canal such that it operates as an internal microphone to detect soundwaves in the ear canal. In other embodiments, the second ear-mounted measurement device may additionally comprise an internal microphone different to the contra-lateral microphone for detecting sound signals in the ear canal. It will be appreciated that any features or configurations described with relation to the first ear-mounted measurement device may be equally applied to corresponding features of the second ear-mounted measurement device.

The controller may be configured to determine an acoustic impedance of the ear drum of the second ear as part of the auxiliary acoustic impedance measurement and compare the acoustic impedance of the ear drum of the first ear determined during the acoustic impedance measurement to the acoustic impedance of the ear drum of the second ear. By providing a basis for comparison of the acoustic impedance measurements or number of eustachian tube openings over a monitoring period, the controller may be able to provide a more accurate diagnosis of the presence or otherwise of eustachian tube dysfunction.

The system may be configured to provide an alert based on the recorded number of eustachian tube openings, or based on the acoustic impedance of the eardrum, changing by more than a predetermined acceptable threshold amount over the monitoring period or a sub-portion of the monitoring period. Alternatively, an alert may be provided based on the number of eustachian tube openings or the acoustic impedance of the ear drum being outside of an expected or acceptable range. For example, the system may provide an alert to a user interface device, which may be remote from the ear-mounted measurement device, if the number of eustachian tube openings or acoustic impedance of the ear drum is below a predetermined minimum threshold. Similarly, the system may be configured to provide an alert to the user interface device if the number of eustachian tube openings or acoustic impedance of the ear drum is above a predetermined maximum threshold. It will be appreciated that it may be the controller which is configured to provide output signalling to cause the alert. The alert may be provided to the user-by-user interface device by way of a visual output, audio output, haptic output or any other suitable sensory output. It will be appreciated that a user-interface device may provide for one or both of receiving an input from a user and providing an output to the user for the user's information. The means for providing the output may be built into the ear-mounted measurement device 2 such as a speaker or alert light built into the ear mounted measurement device. Alternatively, the output may be provided to the user by a user interface device such as by a mobile phone, a tablet computer, a computer, a dedicated alert device or another device. The alert may be provided to the user of the device by way of any of the described methods or it may be provided to a medical practitioner, or it may be provided to both. The alert may only be an alert that an abnormal number of eustachian tube openings or acoustic impedance of the ear drum has been detected or it may be accompanied by data related to the number of eustachian tube openings and/or duration and severity of the abnormal pressure.

In one or more embodiments, the controller may be configured to provide signalling to a user-interface device, which may be a remote from the controller, to cause the user interface device to provide instructions to a user about how to perform a maneuverer. Details on how to perform a maneuverer may include instructions on the action to take or the timing of when to perform the maneuverer. For example, the controller may be configured to provide signalling to cause the user-interface device to indicate to a user how to perform a Valsalva maneuverer, which may be difficult for an inexperienced user to achieve without direction. The instructions to the user about how to perform the maneuverer may be synchronised with an acoustic impedance measurement, a continuous acoustic impedance measurement or a plurality of acoustic impedance measurements of the system in order to provide for improved synchronisation between the action of the maneuverer and the measurement or measurements.

In one or more embodiments, the ear-mounted measurement device 2 may further comprise an internal pressure sensor wherein the internal pressure sensor is arranged to detect the pressure inside the ear canal of the ear to which the ear-mounted measurement device 2 is mounted. The controller may be configured to receive signalling from the pressure sensor indicative of the pressure inside the ear canal and the controller may be configured to determine the acoustic impedance of the ear drum at least partly based on the pressure inside the ear canal over a monitoring period. For example, having a measure of the pressure inside the ear canal may assist in understanding what the acoustic impedance of the ear drum should be and may therefore provide for a more accurate determination of the eustachian tube function.

The system may further comprise an external pressure sensor wherein the external pressure sensor is arranged to detect the ambient pressure around the user. The controller may be configured to receive signalling indicative of the pressure measured by the external pressure sensor. The controller may be configured to perform an acoustic impedance measurement based on the pressure sensed by the external pressure sensor changing by a predetermined amount on in response to the pressure exceeding or reducing below a predetermined threshold value. An eustachian tube opening may be expected when the ambient pressure changes by a predetermined amount or when the ambient pressure goes above or a below one or more predetermined thresholds. Such changes in pressure may be beneficial times to take an acoustic impedance measurement and, in particular, it may be desirable to take one or more acoustic impedance measurements starting before the triggering action and continuing during the measurement or measurements across the triggering action.

The first ear mounted measurement device or the second ear mounted measurement device may comprise an accelerometer wherein the accelerometer is configured to detect a movement of the ear and wherein the controller is configured to trigger an acoustic impedance measurement based on signalling indicative of the movement of the ear from the accelerometer. An accelerometer may be configured to detect any of a plurality of different actions which may cause the triggering of an acoustic impedance measurement which may include a continuous acoustic impedance measurement or a plurality of acoustic impedance measurements over a monitoring period. For example, the accelerometer may be configured to detect, and send signalling to the controller indicative of tilting and swinging of the head. Thus, the accelerometer may also provide for detection of movement that a gyroscope may otherwise provide for. The accelerometer may further detect is the head is moving in a horizontal or vertical direction (with respect to ground) and the acceleration and speed of that movement. This may be particularly helpful, as the accelerometer may be able to detect, for example, if the user is going uphill or if the user is in an elevator. Changes in altitude are known to move a person between areas of different pressure and, further, vertical movement itself can cause changes which require the eustachian tube to open. As such, the controller may be configured to record the detection of a movement of the ear. In some examples, the controller may be configured to continuously record detection of the movement of the ear so that movement of the ear can be correlated with eustachian tube openings. Recording movement of the ear may comprise recording one or more of the direction, speed, tilt and acceleration of the ear.

In one or more embodiments, the controller may be configured to compare movement detected by the accelerometer to one or more movement profiles stored in a movement profile database and, based on identification of a match of the movement to a movement profile, the controller is configured to record the identification of the match. Such measurements of the movement of the ear may provide additional information for determining the functioning of the eustachian tube, particularly when taken in combination with the number of eustachian tube openings over a monitoring time and, for example, when those eustachian tube openings occurred.

Recording the identification of the movement match may comprise recording the fact that a match occurred. Alternatively, or additionally, recording the identification of the movement match may further comprise recording the type of match that occurred. For example, recording a number or frequency of yawn actions over a monitoring period may help in determining the functioning of the eustachian tube in combination with the number of eustachian tube openings. In one or more embodiments, recording the identification of the movement match may further comprise recording the time that the match occurred. The controller may further be configured to correlate the timing of the identification of the movement match with a time when one or more a eustachian tube openings occur. In one or more embodiments, the identification of a match of detected movement to a movement profile may be used in determining the functioning of a eustachian tube.

Further, making one or more acoustic impedance measurement based on such movement may be particularly advantageous. The number of recorded eustachian tube openings over an accelerometer-triggered acoustic impedance measurement may be compared to a corresponding eustachian tube opening movement profile where the eustachian tube opening movement profile is specifically associated with the type of movement being experienced by the user. For example, a different profile may be used for eustachian tube opening comparison when a user is in an elevator compared to when they are standing still.

With reference to figure 3 there is shown schematic examples of plots of ear drum impedance as a function of time illustrating the problem that is solved by the present invention.

Plot I shows a traditional measurement of ear drum impedance where the ear drum impedance is measured at one specific instant of time, whereby there is thus only obtained a "snapshot" of the impedance of the particular ear drum. As the ear drum impedance however may be within the normal range from time to time, and even for periods of time, even though the functioning of the eustachian tube is not normal, a dysfunction of the eustachian tube cannot be detected with the required certainty by performing a traditional ear drum impedance measurement.

This problem is solved by the present invention by monitoring the era drum impedance or a related quantity such as the acoustic admittance or acoustic reflectance of the ear drum over an extended monitoring period as exemplified by plots II, III and IV of figure 3.

Thus, in plot II there is schematically illustrated normal eardrum impedance as a function of time, where the two abrupt changes of eardrum impedance occurs when the eustachian tube opens and equalizes the pressure built-up in the middle ear.

Plot III shows patulous eustachian tube dysfunction i.e., a condition in which the eustachian tube remains open of of the time. The pressure in the middle ear thereby remains constant leading to a constant ear drum impedance.

Plot IV shows the condition in which the eustachian tube remains blocked for a prolonged period of time thereby resulting in a negative pressure and possibly an accumulation of fluid in the middle ear. In this condition, the ear drum impedance increases beyond its normal maximum value as indicated by the shaded region, at the ear drum impedance only returns to normal, when the eustachian tube is finally opened as shown to the right in plot IV.

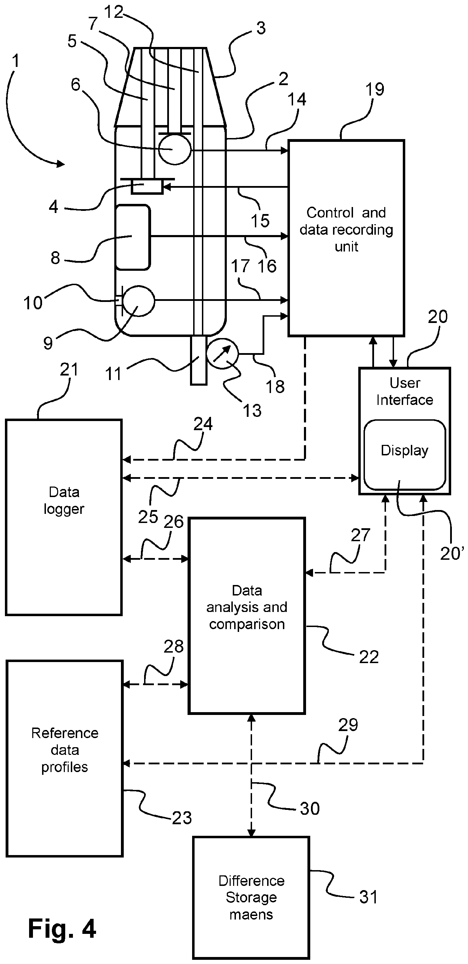

With reference to figure 4 there is shown a block diagram of an example embodiment of a system according to the invention. It is however understood that the various functions represented by the blocks in figure 4 could be implemented differently in practical implementations of the system according to the inventions, such that for instance some of the shown functions could be integrated in a single block.

The system shown in figure 4 comprises the measuring device which comprises an ear-tip 3 formed for insertion into the ear canal of a person. In the housing of the measuring device there is provided a speaker 4 that can emit sound signals to the ear canal via a tube 5. An internal microphone 6 can pick up sound signals from the ear canal via a tube 7. Optionally, the measuring device may comprise a tube 12 through which air pressure can be applied to the ear canal for instance allowing the device to be used in tympanometry. Pressurized aid may be applied through the inlet 11 that may be connected to a pressure gauge 13. The measuring device may optionally be provided by a second microphone 9 that can pick up sound signals from the surroundings via the tube 10. Furthermore 8 an accelerometer may be provided in the measuring device as described above.

Signals and data to and from the microphones, the speaker, the accelerometer and the pressure gauge can pass between the measuring device and the control and data recording unit 19 as indicated by reference numerals 14, 15, 16, 17, 18. It is however understood that the control and data recording unit may form an integral part of the measurement device.

The system according to the shown embodiment further comprises a user interface 20 allowing the user to operate the system. The user interface may be implemented in numerous ways and can for example be a dedicated interface or computer or smart phone or any other similar means. The user interface may typically comprise display means upon which for instance measured ear drum impedance as a function of time can be displayed and compared with target values.

The shown system comprises a datalogging means 21 that for instance can provide the measured data with appropriate identification labels and time information.

The shown system further comprises a data analysis and comparison unit 22 configured to perform data analysis such as averaging of the signals provided by the measuring device. This functional block 22 may also perform comparison of recorded and possibly processed data with corresponding target values or functions that can for instance be provided from a data bank 23 containing reference data profiles. Differences between measured data and target data may for instance be stored in a difference storage means 31.

The required transfer of data between the various functional blocks may take place via transmission paths 24, 25, 26, 27,28, 29 and 30. These transmission paths may be of any appropriate kind, for instance combinations of wired and wireless connections possibly involving the internet or Bluetooth connections. Thus, according to the invention, a geographically distributed system may be set up, enabling for instance the user of a system at a local clinic to download and upload data from and to various remote entities. Also, the system according to the invention enables the performance of different functional blocks to be improved over time by updating software programs in the various functional entities from for instance a central system development unit.

It is understood that the embodiment of a system shown in figure 4 does not constitute a limitation, but that the system according to the invention could be implemented in practice in other manners.

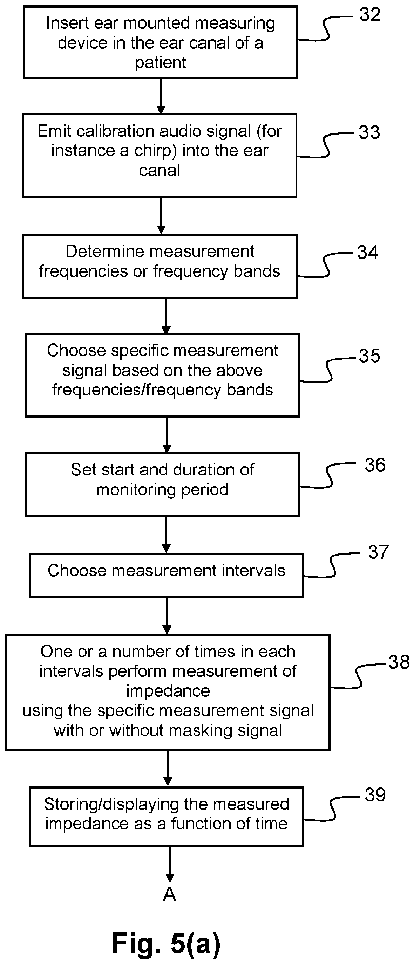

With reference to figure 5 (a) there is shown a flowchart illustrating an embodiment of the method according to the invention. In step 32, the ear mounted measurement device according to the invention is inserted in the ear canal of a patient. As an initial step 33 a calibration audio signal is provided to the ear canal with the primary purpose to determine a frequency or a frequency band that on the particular patient will lead to the best possible determination of the ear drum impedance or related acoustic quantity of this particular patient. The calibration audio signal should cover the whole frequency region in which such preferred frequencies of frequency bands are expected to be located. A suitable audio signal used for calibration purposes can be a chirp covering the whole of said frequency region, but other audio signals may alternatively be used. The calibration process ends by in step 35 choosing a specific measurement audio signal based on the result of step 34 and by in step 36 setting the start of and/or duration of the monitoring period and in step 37 setting chosen measurement intervals. It is understood that additional parameters relating to the measurements taken in the monitoring period could also be specified.

In step 38 the monitoring of the ear drum impedance or related acoustic quantities is carried out, for for instance one or a number of times in each interval to perform the ear drum impedance measurement or similar measurement. This can, as described above, be carried out with or without the application of an audio masking signal.

In step 39, the method as shown in figure 5(a) ends by storing and/or displaying the measured ear drum impedance or related acoustic quantity. A medically trained person may thus examine the stored/displayed measurement result over the monitoring period and this result may help him forming his opinion on the functioning of the middle ear and/or eustachian tube.

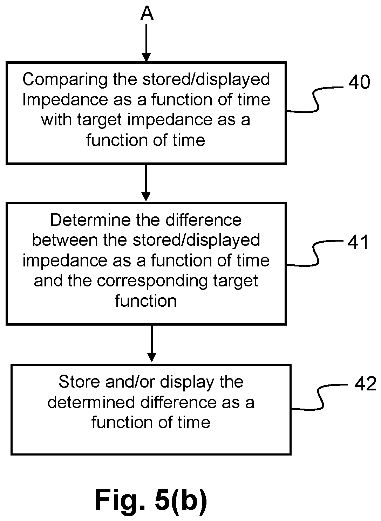

The method according to the embodiment shown in figure 5(a) may continue with the steps shown in figure 5(b).

In step 40 the stored and/or displayed ear drum impedance or similar quantity as a function of time is compared with one or more corresponding target functions of ear drum impedance or other related acoustic quantity over time. In step 41 the difference between the measured results and corresponding target functions is determined and this difference is in step 42 displayed and/or stored.

It is understood that the flow charts shown in figures 5(a) and 5(b) are only examples of the method according to the invention and that the method according to the invention may comprise numerous other steps, which would appear from the disclosure of the invention and the previous paragraphs of the detailed description of the invention.

## Claims

1. A system comprising a measurement device wherein the measurement device is configured to be mounted to the ear of a user, the user's ear having an ear canal and an ear drum, the measurement device comprising:
- a speaker (4) configured to emit a sound signal into the ear canal as part of a measurement of the acoustic impedance or a related quantity such as the acoustic admittance or reflectance of the ear drum;
- an internal microphone (6) configured to detect sound signals in the ear canal; and
- a securing apparatus configured to provide for securing of the measurement device to the ear,
wherein the system further comprises a controller (19) configured to measure and monitor the acoustic impedance or a related acoustic quantity such as the acoustic admittance or reflectance of the ear drum as a function of time over a monitoring period based on a measurement sound signal emitted by the speaker (4) and a corresponding sound signal detected by the microphone (6), and display means (20') configured to display the acoustic impedance or related acoustic quantity as a function of time.

2. A system according to claim 1, where the system is provided with storage means configured to store one or more target acoustic impedance or related acoustic quantity as a function of time, and during the monitoring period comparing the measured acoustic impedance or related acoustic quantity as a function of time with a corresponding target acoustic impedance or related acoustic quantity as a function of time and determining the difference (22) between the determined acoustic impedance or related acoustic quantity as a function of time and the corresponding target (23) as a function of time and displaying (20) and/or storing (30) said difference as a function of time on suitable display means (20') and/or in suitable storage means (30).

3. A system according to claim 1 or 2, wherein the system comprises means for providing a calibration audio signal to the ear drum, where the calibration audio signal is configured to allow determining a measurement frequency or measurement frequency band, which is suitable for determining the acoustic impedance or the related acoustic quantity.

4. A system according to claim 3, wherein said calibration audio signal is a broadband audio signal such as a chirp covering the frequency range in which the acoustic impedance or related acoustic quantity is to be measured.

5. A system according to any of the preceding claims, wherein the system comprises means for providing a masking audio signal, the acoustic characteristics of which enables it to wholly or partially mask the measurement audio signal, and where the system during the monitoring period in addition to the measurement audio signal at the measurement frequency band or measurement frequency band also emits the masking audio signal, such that a person whose ear drum characteristics are being monitored during the monitoring period is less annoyed by the measurement signal and hence will be more able to accept a prolonged monitoring period.

6. A system according to any of the preceding claims, wherein said controller is configured to interpret changes of the acoustic impedance or related acoustic quantity of the ear drum as an opening of the eustachian tube and the controller is configured to record the number of eustachian tube openings over the monitoring period.

7. A system according to claim 6 wherein the controller is further configured to determine one or more additional characteristics of the opening of the eustachian tube based on the changes of the acoustic impedance or said related acoustic quantity of the ear drum, wherein the one or more additional characteristics of the opening of the eustachian tube comprises one or more of:
a duration of a eustachian tube opening;
a time between eustachian tube openings; and
the shape of the plot of eustachian tube opening as a function of time for instance in order to ascertain that the plot in fact represents an opening of the eustachian tube and not for instance random noise.

8. A system according to any of the preceding claims, wherein the system is configured to monitor the acoustic impedance or said related quantities of the ear drum over the monitoring period by being configured to one or more of:
emit a sound signal continuously over at least a portion of the monitoring period, wherein the continuously emitted sound signal is emitted for a sufficient duration to resolve at least two measurements of the acoustic impedance of the ear drum;
emit a plurality of successive sound signals over at least a portion of the monitoring period having a predetermined delay period between each successive emitted sound signal;
and wherein the controller is configured to one or more of:
continuously determine the acoustic impedance or said related quantity of the ear drum for long enough to resolve at least two measurements of the acoustic impedance or said related quantity of the eardrum; or
determine the acoustic impedance or said related quantity of the eardrum a plurality of individual times, wherein each determination of the acoustic impedance or said related quantity is based on the reflections of successively emitted sound signals having a predetermined delay period therebetween.

9. A system according to any of the preceding claims wherein the system is configured to provide an alert based on the acoustic impedance or said related quantity of the ear drum changing by more than a predetermined acceptable threshold amount.

10. A system according to any of the preceding claims wherein the controller is configured to compare an audio signal detected by the internal microphone to one or more audio signal profiles stored in an audio signal profile database and, based on identification of a match of the detected audio signal to an audio signal profile, the controller records an identification of the match, wherein the audio signal profiles of the audio signal profile database correspond to one or more of the list comprising, but not limited to: a yawn action; a swallow action; a breathing action; a coughing action, a Toynbee maneuverer or a Valsalva maneuverer.

11. A system according to any of the preceding claims wherein the ear-mounted measurement device further comprises an external microphone configured to detect audio signals in the environment around the user and wherein the controller is configured to use the audio signals detected by the external microphone to compensate for audio signals detected by the internal microphone originating external to the ear canal.

12. A system of any preceding claim wherein the ear-mounted measurement device further comprises a sensor configured to detect the orientation and/or movement of the body and/or head of the person whose ear drum impedance or relates acoustic quantity is being measured.

13. An ear-mounted measurement device configured to be mounted to the ear of a user, the user's ear having an ear canal and an ear drum, the ear-mounted measurement device comprising at least:
a speaker configured to emit a soundwave at a measurement frequency into the ear canal as part of an acoustic impedance measurement;
an internal microphone configured to detect soundwaves in the ear canal; and
a securing apparatus configured to provide for securing of the ear-mounted measurement device to the ear,
wherein the ear-mounted measurement device is configured to provide signalling to a controller indicative of the soundwaves detected by the internal microphone for recording the number of eustachian tube openings over a monitoring by monitoring the acoustic impedance of the ear drum over the monitoring period.

14. A controller configured to:
provide signalling to an ear-mounted measurement device or a system to cause a speaker of the ear-mounted measurement device to emit a soundwave at a measurement frequency as part of an acoustic impedance measurement;
receive signalling from a microphone of the ear-mounted measurement device indicative of audio detected by the microphone;
monitor the acoustic impedance of the ear drum over a monitoring period based on the signalling indicative of the audio detected by the microphone as part of the acoustic impedance measurement wherein changes of the acoustic impedance of the ear drum are indicative of the opening of the eustachian tube; and
record the number of eustachian tube openings over the monitoring period.

15. A method for measuring the acoustic impedance or a related acoustic quantity such as the acoustic admittance or reflectance of the ear drum of a patient as a function of time over a monitoring period, the method comprising the steps of:
- providing an ear-mounted measurement device configured to be mounted to the ear of a user, the user's ear having an ear canal and an ear drum, the ear-mounted measurement device comprising at least a speaker configured to emit a sound signal into the ear canal and an internal microphone configured to detect sound signals in the ear canal;
- via the ear canal providing a calibration audio signal to the ear drum, where the calibration audio signal is configured to allow determining an individual measurement frequency or measurement frequency band which is suitable or preferably optimal for determining the acoustic impedance or the related acoustic quantity;
- during a monitoring period, providing a measurement sound signal at the individual measurement frequency or frequency band to the ear canal of the patient a plurality of times during the monitoring period, whereby the acoustic impedance or related acoustic quantity is measured a corresponding plurality of times during the monitoring period;
- storing and/or displaying the measured acoustic impedance or related acoustic quantity as a function of time.

16. A method according to claim 15, wherein said calibration audio signal is a chirp covering the frequency range in which the acoustic impedance or related acoustic quantity is to be made.

17. A method according to claim 15 or 16, wherein said speaker in the ear-mounted measurement device during the monitoring period in addition to the signal at the measurement frequency band or measurement frequency band also emits an audio signal, the acoustic characteristics of which enables it to wholly or partially mask the measurement audio signal.
